(19) Europäisches Patentamt
European Patent Office
Office européen des brevets

(11) **EP 2 926 845 B1**

(12) **EUROPÄISCHE PATENTSCHRIFT**

(45) Veröffentlichungstag und Bekanntmachung des Hinweises auf die Patenterteilung:
**20.12.2017 Patentblatt 2017/51**

(51) Int Cl.:
***A61M 1/16*** *(2006.01)* ***G01N 33/487*** *(2006.01)*

(21) Anmeldenummer: **15158666.6**

(22) Anmeldetag: **11.03.2015**

(54) **Vorrichtung zum Bestimmen eines Verteilungsvolumens bei einem Dialysepatienten**

Device for determining a volume of distribution for a dialysis patient

Dispositif de détermination d'un volume de distribution d'un patient dialysé

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.04.2014 DE 102014104768**

(43) Veröffentlichungstag der Anmeldung:
**07.10.2015 Patentblatt 2015/41**

(73) Patentinhaber: **B. Braun Avitum AG**
**34212 Melsungen (DE)**

(72) Erfinder:
• **Wolff, Henrik**
**37213 Witzenhausen (DE)**

• **Meibaum, Jörn**
**34225 Baunatal (DE)**
• **Strohhöfer, Dr. Christof**
**34123 Kassel (DE)**

(74) Vertreter: **Winter, Brandl, Fürniss, Hübner, Röss, Kaiser, Polte - Partnerschaft mbB Patent- und Rechtsanwaltskanzlei**
**Alois-Steinecker-Strasse 22**
**85354 Freising (DE)**

(56) Entgegenhaltungen:
**EP-A1- 1 698 360     WO-A1-98/55166**
**US-B1- 7 077 819**

**EP 2 926 845 B1**

## Beschreibung

Die Erfindung betrifft eine Vorrichtung und ein Verfahren zum Bestimmen eines Verteilungsvolumens wenigstens einer urämischen Substanz bei einem Dialysepatienten.

In der dialysefreien Zeit sammelt sich neben urämischen Substanzen ein zusätzliches Flüssigkeitsvolumen im Dialysepatienten an. Neben dem Entfernen urämischer Substanzen ist Aufgabe der Dialysetherapie diese Flüssigkeitsmenge zu entziehen. Dieser Prozess wird als Ultrafiltration bezeichnet. Die zu entziehende Flüssigkeitsmenge ergibt sich aus der Differenz des Patientengewichts zu Beginn der Dialysebehandlung und einem ärztlich bestimmten Trockengewicht, dem Gewicht des Patienten ohne Überwässerung. Das Flüssigkeitsvolumen des Körpers, in dem sich Harnstoff, der prominenteste Vertreter kleinmolekularer harnpflichtiger Substanzen, verteilt, wird als Harnstoffverteilungsvolumen bezeichnet. Das Harnstoffverteilungsvolumen ist annähernd gleich dem Körperwasservolumen. Zu Beginn der Behandlung setzt sich dieses Volumen aus dem "Trockenvolumen", dem Flüssigkeitsvolumen eines Dialysepatienten bei dem ärztlich ermittelten Trockengewicht, und dem in der dialysefreien Zeit angesammelten Volumen zusammen. Es ist also um den gleichen Wert erhöht wie das Trockengewicht des Patienten. Somit ist das Harnstoffverteilungsvolumen ein wichtiger Parameter zur Überwachung des Flüssigkeitsstatus und des Allgemeinzustands des Patienten. Für eine Bestimmung des Harnstoffverteilungsvolumens während der Behandlung fehlen derzeit geeignete Messmethoden.

Für die Beurteilung des Entwässerungs-Ergebnisses während der Behandlung stehen mehrere Verfahren zur Verfügung. Vielfach werden Bioimpedanzmessungen eingesetzt, die mittels Analyse der "Körperimpedanz" bei typischerweise 50 kHz den Fluidstatus des Patienten überwachen. Aus US 2007/0027402 A1 ist ein Bioimpedanzmessverfahren bekannt, bei dem ein Wechselstrom mit mehreren Frequenzen durch wenigstens zwei Punkte auf der Haut geleitet wird, für jede Frequenz der Spannungsabfall an wenigstens zwei weiteren Punkten erfasst wird, und die aufgezeichneten Spannungsabfallwerte für verschiedene Frequenzen ausgewertet werden, um wenigstens einen numerischen Wert für Muskel, Fett, extrazelluläres Fluid abzuleiten.

Aus WO 2010/043381 A2 ist die Überwachung des Hydrations- und/oder Ernährungszustandes eines Patienten mit Hilfe von Bioimpedanzmesswerten bekannt. Das entsprechende Verfahren umfasst die Schritte: Messen von Bioimpedanzdaten am Patienten zum Zeitpunkt t, Bestimmen eines Volumenkompartiments des Patienten anhand der gemessenen Bioimpedanzdaten, Bestimmen des korrigierten Volumenkompartiments durch Multiplikation des Volumenkompartiments oder einer damit korrelierten Größe mit einem für das fehlende Gliedmaß oder Teile davon charakteristischen Faktor k.

Allerdings ist die Messung mittels Bioimpedanzmessung aufwändig und erfordert zusätzliche Messeinrichtungen, welche zudem kostenintensiv sind.

Ein weiteres Verfahren verwendet die Massen- und Konzentrationsbestimmung von Substanzen, bei der sich das Verteilungsvolumen V quasi als Nebenprodukt aus Masse und Konzentration der gesuchten Substanz gemäß $V = m / c$ ergibt, wobei m die Masse in g und c die Konzentration in g/ml ist.

Aus EP 1 037 681 B1 ist die Bestimmung der Plasmakonzentration über die Dialysierflüssigkeitskonzentration bekannt. Die Bestimmung von V erfolgt über die Gesamtmasse an Harnstoff im Patienten.

Aus EP 0 986 410 B1 ist die Berechnung der Gesamtkörpermasse eines gelösten Stoffes in einem Verteilungsvolumen bekannt.

Ferner ist aus DE 698 34 034 T2 eine Vorrichtung zum Berechnen einer Gesamtkörpermasse eines gelösten Stoffes in einem Flüssigkeitsvolumen bekannt.

Ein weiteres Verfahren für die Beurteilung des Entwässerungs-Ergebnisses während der Behandlung beruht auf der Überwachung des relativen Blutvolumens, speziell nach Therapieende. Dies ist beschrieben u.a. in Lopot et al.: "Continuous blood volume monitoring and dry weight assessment", J Ren Care 2007 Apr-Jun; 33(2) 52-8, und Rodriguez et al.: "Assessment of dry weight by monitoring changes in blood volume during hemodialysis using Crit-Line", Kidney International (2005) 68, 854-861.

Ein Nachteil der Bioimpedanzmessung besteht darin, dass eine spezielle Messtechnik benötigt wird, die nicht in die Dialysemaschine integriert ist. Bei der Berechnung der Konzentration oder Masse der ausgewaschenen Substanzen muss die Gesamtmasse der im Flüssigkeitsvolumen des Körpers gelösten Substanzen einbezogen werden. Die Gesamtmasse im Körper ist allerdings ein schwer zugänglicher Parameter. WO98/55166 A1 offenbart eine Vorrichtung zum Berechnen von der Dialyseeffizienz. Aus US7077819 B1 ist eine Vorrichtung zum Bestimmen des Harnstoffverteilungsvolumens bekannt. Es ist die Aufgabe der Erfindung, eine Vorrichtung zu schaffen und ein Verfahren anzugeben, womit sich das Verteilungsvolumen von Harnstoff im Körper des Patienten verlässlich abschätzen lässt und der Verlauf und das Ergebnis der Dialyse beurteilt werden kann.

Diese Aufgabe wird gelöst durch die Vorrichtung nach Anspruch 1. Bevorzugte Ausführungsformen der Erfindung sind Gegenstand der jeweiligen Unteransprüche.

Der Erfindung liegt die Überlegung zugrunde, dass sich bei Kenntnis der Dialysedosis D, dem Reinigungsgrad ("Clearance") K und der Dialysedauer t das gesuchte Verteilungsvolumen in einfacher Weise aus der Gleichung

$$D = K * t / V$$

berechnen lässt, wobei D die Dialysedosis [dimensionslos], K der Reinigungsgrad [ml/min], V das zu reinigende Volumen [ml] und t die Dialysedauer [min] ist. Der Reinigungsgrad K wird im folgenden auch als "Clearance" oder Clearance-Faktor bezeichnet.

Diese Berechnung lässt sich insbesondere "online", d.h. während der Therapie, durchführen, so dass sich zu jedem Zeitpunkt das Harnstoffverteilungsvolumen ermitteln lässt. Die Dialysedosis D = K * t /V kann mit den heute bekannten und verwendeten Verfahren "online" oder "quasi-online" ermittelt werden. Die dafür zur Verfügung stehenden Verfahren sind die Bestimmung von D durch Leitfähigkeitsmessung, die Bestimmung von D durch chemische Reaktionen (Urease), die Bestimmung von D durch optische Absorptionsmessungen oder die Bestimmung von D durch Blutprobenentnahme.

Diese Aufzählung der Verfahren zur Bestimmung der Dialysedosis D ist nicht abschließend, und es können weitere Verfahren eingesetzt werden, welche die Bestimmung der Dialysedosis D unter den genannten Bedingungen ermöglichen.

Die erfindungsgemäße Vorrichtung zum Bestimmen eines Verteilungsvolumens wenigstens einer urämischen Substanz bei einem Dialysepatienten ist versehen mit:

- einem Dialysator zum Entziehen der wenigstens einen Substanz aus einer den Dialysator durchströmenden Körperflüssigkeit, wobei die wenigstens eine entzogene Substanz über eine den Dialysator ebenfalls durchströmende Dialysierflüssigkeit entsorgt wird,
- optional einer Flussraten - Messeinrichtung zum Erfassen einer Durchflussrate der Dialysierflüssigkeit durch den Dialysator,
- einer Dialysierflüssigkeit - Messeinrichtung zum Erfassen wenigstens eines Dialysierflüssigkeit - Parameters, der von der wenigstens einen Substanz in der Dialysierflüssigkeit abhängt,

und ist dadurch gekennzeichnet, dass

- mindestens eine Rechnereinrichtung vorgesehen ist zum Ermitteln einer Dialysedosis aus dem Dialysierflüssigkeit - Parameter und zum Ermitteln des Verteilungsvolumens unter Berücksichtigung eines Clearance-Faktors (d.h. des Reinigungsgrads) und einer Dialysedauer.

Bevorzugte Ausführungsformen der erfindungsgemäßen Vorrichtung weisen einzeln oder in Kombination als weitere Merkmale auf, dass

- die Rechnereinrichtung die Dialysedosis aus einer gemessenen optischen Extinktion der Dialysierflüssigkeit ermittelt;
- die Rechnereinrichtung die Dialysedosis aus einer gemessenen Leitfähigkeit der Dialysierflüssigkeit ermittelt;
- die Rechnereinrichtung die Dialysedosis aus einer erfassten chemischen Reaktion (Urease) ermittelt;
- die Rechnereinrichtung die Dialysedosis in Abhängigkeit von dem Dialysierflüssigkeit - Parameter zu einem ersten Zeitpunkt t1 und wenigstens zu einem zweiten Zeitpunkt t2 ermittelt;
- die Rechnereinrichtung die Dialysedosis als Mittelwert von wenigstens zwei Dialysedosis - Werten ermittelt;
- die Rechnereinrichtung die Dialysedosis in Abhängigkeit von dem Dialysierflüssigkeit - Parameter und von einem Flüssigkeitsentzug des Dialysepatienten ermittelt;
- die Dialysierflüssigkeit - Messeinrichtung den Dialysierflüssigkeit - Parameter und die Flussraten - Messeinrichtung den Flüssigkeitsentzug zu wenigstens einem ersten Zeitpunkt t1 und einem zweiten Zeitpunkt t2 erfasst und die Rechnereinrichtung das Verteilungsvolumen durch Vergleich der Dialysierflüssigkeit - Parameter, des Flüssigkeitsentzugs und der Zeitdauer zwischen t1 und t2 ermittelt;
- die Rechnereinrichtung die Werte für den Dialysierflüssigkeit - Parameter und den Flüssigkeitsentzug zu dem wenigstens ersten und zweiten Zeitpunkt mit jeweils einem vorgegebenen Wert wichtet.

Das Verfahren zum Bestimmen eines Verteilungsvolumens wenigstens einer urämischen Substanz bei einem Dialysepatienten weist die Schritte auf:

- Entziehen der wenigstens einen Substanz aus einer Körperflüssigkeit mittels eines von der Körperflüssigkeit durchströmten Dialysators und Entsorgen der wenigstens einen Substanz mittels einer den Dialysator ebenfalls durchströmenden Dialysierflüssigkeit

- optional Erfassen einer Durchflussrate der Dialysierflüssigkeit durch den Dialysator mittels einer Flussraten - Messeinrichtung,
- Erfassen wenigstens eines Dialysierflüssigkeit - Parameters, der von der wenigstens einen Substanz in der Dialysierflüssigkeit abhängt, mittels einer Dialysierflüssigkeit - Messeinrichtung,
- Ermitteln einer Dialysedosis aus dem Dialysierflüssigkeit - Parameter mittels mindestens einer Rechnereinrichtung und Ermitteln des Verteilungsvolumens aus der Dialysedosis unter Berücksichtigung eines Clearance-Faktors und einer Dialysedauer mittels der Rechnereinrichtung.

Bevorzugte Ausführungsformen des Verfahrens weisen einzeln oder in Kombination als weitere Merkmale auf, dass

- die Dialysedosis mittels der Rechnereinrichtung aus einer gemessenen optischen Extinktion der Dialysierflüssigkeit ermittelt wird;
- die Dialysedosis mittels der Rechnereinrichtung aus einer gemessenen Leitfähigkeit der Dialysierflüssigkeit ermittelt wird;
- die Dialysedosis mittels der Rechnereinrichtung aus einer erfassten chemischen Reaktion (Urease) ermittelt wird;
- die Dialysedosis mittels der Rechnereinrichtung in Abhängigkeit von dem Dialysierflüssigkeit - Parameter zu einem ersten Zeitpunkt t1 und wenigstens zu einem zweiten Zeitpunkt t2 ermittelt wird;
- die Dialysedosis mittels der Rechnereinrichtung als Mittelwert von wenigstens zwei Dialysedosis-Werten ermittelt wird;
- die Dialysedosis mittels der Rechnereinrichtung in Abhängigkeit von dem Dialysierflüssigkeit - Parameter und von einem Flüssigkeitsentzug des Dialysepatienten ermittelt wird;
- der Dialysierflüssigkeit - Parameter mittels der Dialysierflüssigkeit - Messeinrichtung und der Flüssigkeitsentzug mittels der Flussraten - Messeinrichtung zu wenigstens einem ersten Zeitpunkt t1 und einem zweiten Zeitpunkt t2 erfasst wird und die Dialysedosis mittels einer Rechnereinrichtung durch Vergleich der Dialysierflüssigkeit - Parameter, des Flüssigkeitsentzugs und der Zeitdauer zwischen t1 und t2 ermittelt wird;
- die Werte für den Dialysierflüssigkeit - Parameter und den Flüssigkeitsentzug mittels der Rechnereinrichtung zu dem wenigstens ersten und zweiten Zeitpunkt mit jeweils einem vorgegebenen Wert gewichtet werden.

Die Erfindung hat u.a. den Vorteil, dass durch die unverzögerte, sog. "online"-Bestimmung der Dialysedosis D zu jedem Zeitpunkt während der Therapie das aktuelle Harnstoffverteilungsvolumen des Patienten bestimmt werden kann. Neben der Überwachung von Parameter-Änderungen während der Therapie können darüber hinaus die Werte zu Therapiebeginn und Ende analysiert werden, um die Änderungen in der dialysefreien Zeit zu charakterisieren. Außerdem ist kein zusätzlicher Messaufbau notwendig, und das Problem der schwer zugänglichen Gesamtmasse gelöster Substanzen wird umgangen.

Weitere Merkmale und Vorteile der Erfindung ergeben sich aus der folgenden Beschreibung von bevorzugten Ausführungsformen der Erfindung.

Die erfindungsgemäße Bestimmung des Harnstoffverteilungsvolumens beruht darauf, dass die Dialysedosis D sowie die sog. Clearance des Dialysators K und die Dauer der Dialyse bzw. das Zeitintervall oder der Messzeitraum t bekannt sind. Die Clearance kann während der Dialyse unverzögert bestimmt werden oder aus einem Speicher ausgelesen werden.

Für die erfindungsgemäße Bestimmung des Harnstoffverteilungsvolumens ist es wichtig, dass die Bestimmung der Dialysedosis D ohne implizite Modellannahmen auskommt, d.h. dass insbesondere kein Verteilungsvolumen theoretisch vorausgesetzt wird, um die Dialysedosis D zu bestimmen.

Im folgenden wird die erfindungsgemäße Vorrichtung anhand von mehreren Ausführungsbeispielen unter Bezugnahme auf die Prinzipskizze gemäß der einzigen Figur erläutert.

Verfahren 1

Es wird angenommen, dass eine Dialysedosis D von 0,7 nach einer Therapiezeit von 120 min vorliegt. Die Clearance K beträgt 285 ml/min. Somit lässt sich das Verteilungsvolumen V bestimmen durch:

$$K * t / V = 0{,}7 = (0{,}285 \text{ l/min} * 120 \text{ min}) / V$$

$$V = 48{,}9 \text{ l.}$$

Je größer der Messzeitraum t wird, desto weniger ist die Clearance K als konstant anzunehmen. Wird von einer Änderung der Clearance K während des Messzeitraums t ausgegangen, kann dies über eine Mittelwertbildung über den Messzeitraum t kompensiert werden.

Bei der Dialyse handelt es sich in der Regel um eine Kombination aus Hämodialyse und Hämofiltration, bei der dem Patienten auch Flüssigkeit über den Dialysator entzogen wird. Das bedeutet, dass sich während des Messzeitraums t das Verteilungsvolumen V der urämischen Substanzen durch den Flüssigkeitsentzug ändert, was das Ergebnis der obigen Berechnung verfälscht. Dies kann kompensiert werden, indem der Messzeitraum t klein gehalten wird und somit der Flüssigkeitsentzug zu vernachlässigen ist. Ist die Vernachlässigung des Flüssigkeitsentzugs nicht mehr möglich, muss der Flüssigkeitsentzug UF (Ultrafiltration) in der Berechnung Berücksichtigung finden. Die obige Gleichung wird dann wie folgt modifiziert:

$$K * t / (0{,}5 * [V1 + \{V1 - UF\}]) = 0{,}7 = (0{,}285 \ l/min * 120 \ min) / (V1 - 0{,}5 * UF)$$

$$V1 = 48{,}9 \ l + 0{,}5*UF$$

oder

$$K * t / (0{,}5 * [V2 + \{V2 + UF\}]) = 0{,}7 = (0{,}285 \ l/min * 120 \ min) / (V2 + 0{,}5 * UF)$$

$$V2 = 48{,}9 \ l - 0{,}5 * UF.$$

V1 ist das Volumen zu Beginn des Messzeitraums, V2 das Volumen am Ende des Messzeitraums. Wird V2 zu Therapieende bestimmt, entspricht es dem Verteilungsvolumen, das bei Trockengewicht vorliegt, und kann als Kontrollgröße verwendet werden.

In einer Variante des Verfahrens 1 kann bei der unverzögerten Bestimmung der Dialysedosis D der Quotient K / V auch direkt ermittelt werden. Dieser Quotient kann ebenfalls zur Bestimmung des Harnstoffverteilungsvolumens verwendet werden. Dies führt zu folgenden zwei Gleichungen zur Bestimmung des Verteilungsvolumens V:

$$K / V = 0{,}0058 \ 1/min = (0{,}285 \ l/min) / V$$

$$V = 49{,}1 \ l.$$

Verfahren 2

Aus einem Dialysierflüssigkeit - Parameter, aus dem die Dialysedosis ermittelt wird, kann durch wiederholte Aufnahme im Abstand einiger Minuten ein Gleichungssystem mit zwei Gleichungen erstellt werden. Formell gelten Clearance K und Verteilungsvolumen V in beiden Gleichungen als Unbekannte. Da sich das Körpervolumen des Patienten abgesehen von dem Entzug durch Ultrafiltration nicht ändert und die Clearance K in dem genannten Zeitraum als konstant angenommen wird, sind diese Unbekannten in beiden Gleichungen identisch. Somit lässt sich durch das Aufnehmen zweier Messpunkte für den Wert für die Dialysedosis D das Verteilungsvolumen V bestimmen. Man eliminiert die Unbekannte K durch Einsetzen und bestimmt V durch Auflösen. X und Y stellen dabei Messwerte für die Dialysedosis D dar:

$$K * t1 / V1 = X \qquad (i)$$

$$K * t2 / V2 = Y \qquad (ii)$$

$$=> Y * (V2 / V1) * (t1 / t2) = X$$

mit V2 = V1 - UFR * $\Delta$t, wobei $\Delta$t = |t1 - t2| gilt. Die UFR ist hierbei die mittels der Flussraten - Messeinrichtung ermittelte Ultrafiltrationsrate in ml/min und t die Zeit in min. Die Flussraten - Messeinrichtung kann dabei von beliebiger Gestallt und Ausführung sein. Es kann sich beispielsweise um einen invasive oder nicht invasiven Flussmeser handeln. Auch reicht es, die Förderpumpe selbst als Flussraten-Messeinrichtung einzusetzen, aus der sich das geförderte Volumen pro Zeit ermitteln lässt.

Durch Umstellen der Gleichungen folgt:

$$=> Y * t1 / t2 * V1 - Y * t1 / t2 * UFR * \Delta t - X * V1 = 0$$

$$V1 = ((Y * t1 / t2) / (t1 / t2 * Y - X)) \; UFR * \Delta t.$$

Um die Genauigkeit der Berechnung zu erhöhen und die Bestimmung des Harnstoffverteilungsvolumens unabhängig von Schwankungen des Messsignals zu machen, können statt zwei Gleichungen auch mehrere Gleichungen zu Zeitpunkten t1 ... tn verwendet werden. Die Zeitpunkte t1 ... tn liegen dabei vorzugsweise nicht zu weit auseinander, so dass die Clearance K als konstant angenommen werden kann. Aus allen Kombinationen der Gleichungen 1 ... n wird das Harnstoffverteilungsvolumen ermittelt. Die Ergebnisse werden dann gemittelt, vorzugsweise gewichtet, um zu einem endgültigen Wert V für das Harnstoffverteilungsvolumen zu kommen.

In einer Variante des Verfahrens 2 wird das Verteilungsvolumen V unter Verwendung des Quotienten K / V ermittelt. Bestimmt man den Quotienten K / V zu zwei verschiedenen Zeitpunkten, die jedoch nicht zu weit auseinander liegen, so kann mit Hilfe der Werte A und B das Körperverteilungsvolumen V unter Berücksichtigung der Ultrafiltration berechnet werden. Die Zeitpunkte t1 bzw. t2 müssen nur noch zur Bestimmung der entzogenen Flüssigkeitsmenge verwendet werden. Dies ist in den folgenden Gleichungen (iii) und (iv) dargestellt:

$$K / V1 = A \qquad (iii)$$

$$K / V2 = B \qquad (iv)$$

mit V2 = V1 - UFR * $\Delta$t, wobei $\Delta$t = |t1 - t2| gilt.

Durch Umstellen der Gleichungen folgt:

$$=> V1 = (-B * UFR * \Delta t) / (A - B).$$

Das folgende Beispiel bezieht sich auf das Messverfahren nach Gleichung (i) und (ii), kann aber ebenso mit dem vereinfachten Verfahren nach Gleichung (iii) und (iv) durchgeführt werden.

$$t1 = 20 \; min; \; K * t / V = 0{,}1000 = X$$

$$t2 = t1 + 20 \; min; \; K * t / V = 0{,}2008 = Y$$

$$\Delta t = 20 \text{min}$$

$$UFR = 1/2 \ l/h = 1/120 \ l/min$$

$$\Rightarrow V1 = 0{,}2003 * (20 / 40) / (0{,}2003 * (20 / 40) - 0{,}10000) * 1 / 120 * 20 = 41{,}83 \ l.$$

Mit diesen Ausführungsformen ist eine verzögerungsfreie Überwachung des Behandlungsverlaufs zur verzögerungsfreien Ermittlung des aktuellen Fluidstatus möglich. Es ist, anders als im Stand der Technik, kein zusätzlicher Messaufbau notwendig und das Problem der schwer zugänglichen Gesamtmasse gelöster Substanzen wird umgangen. Durch die verzögerungsfreie Bestimmung der Dialysedosis D kann zu jedem Zeitpunkt während der Therapie das aktuelle Harnstoffverteilungsvolumen des Patienten bestimmt werden.

Es ist darüber hinaus möglich, die Verteilungsvolumina anderer Substanzen wie z.B. Harnsäure, Creatinin, Phosphat, beta2-Mikroglobulin, Indoxylsulfat, p-Cresol oder p-Cresylsulfat zu bestimmen. Deren Verteilungsvolumina weichen vom Gesamtflüssigkeitsvolumen ab, was ihre eingeschränkte Verteilung und Diffusivität im Körper widerspiegelt. Die Kenntnis dieser Verteilungsvolumina dient primär nicht dazu, den Flüssigkeitsstatus des Patienten zu bestimmen. Sie kann aber dazu dienen, den Reinigungsprozess der jeweiligen Substanz und speziell das stoffspezifische, körperinterne Verhalten besser zu beschreiben und so die Behandlung gezielter zu optimieren.

Die Vorrichtung zum Umsetzen des Verfahrens, mit der sich das Verteilungsvolumen wenigstens einer urämischen Substanz bei einem Dialysepatienten bestimmen lässt, umfasst gemäß der anliegenden Figur die im folgenden kurz dargestellten Komponenten.

Ein Dialysator ist vorgesehen, mit dem sich wenigstens eine Substanz aus einer Körperflüssigkeit (z.B. Blut) des Patienten entziehen und über eine Dialysierflüssigkeit entsorgen lässt. Der Dialysator d.h. insbesondere seine Zu- und/oder Abläufe können optional mit einer Flussraten - Messeinrichtung versehen sein, um eine Durchflussrate der Dialysierflüssigkeit durch den Dialysator zu erfassen. Darüber hinaus wird durch eine Dialysierflüssigkeit - Messeinrichtung wenigstens ein Dialysierflüssigkeit - Parameter erfasst, der für die wenigstens eine Substanz in der Dialysierflüssigkeit charakteristisch ist.

Mit der Rechnereinrichtung wird aus dem Dialysierflüssigkeit - Parameter die Dialysedosis D ermittelt. Die Rechnereinrichtung ermittelt dann aus der Dialysedosis das Verteilungsvolumen, wobei der Clearance-Faktor K und die Dialysedauer t in die Berechnung eingehen, wie vorstehend erläutert wurde.

Die Ermittlung der Dialysedosis mittels der Rechnereinrichtung kann auf die nachfolgend aufgeführten Arten erfolgen. Eine erste Art der Ermittlung der Dialysedosis basiert auf der Messung der optischen Extinktion der Dialysierflüssigkeit. Eine zweite Art der Ermittlung der Dialysedosis basiert auf der Messung der Leitfähigkeit der Dialysierflüssigkeit. Eine dritte Art der Ermittlung der Dialysedosis ist basiert auf der Erfassung einer entsprechenden chemischen Reaktion (Urease).

Um die Genauigkeit und Zuverlässigkeit der Volumenbestimmung zu verbessern, kann der Dialysierflüssigkeit - Parameter mehrmals erfasst und daraus die Dialysedosis mehrmals ermittelt werden, d.h. zu einem ersten Zeitpunkt t1 und wenigstens zu einem zweiten Zeitpunkt t2. Selbstverständlich können auch weitere Messungen erfolgen. Die Dialysedosis wird dann als Mittelwert der Dialysedosen aus den wenigstens zwei Dialysierflüssigkeit - Parameter - Werten ermittelt.

Unabhängig von der Mehrfacherfassung des Dialysierflüssigkeit - Parameters kann bei der Ermittlung der Dialysedosis auch der Flüssigkeitsentzug des Dialysepatienten berücksichtigt werden. Dazu fließt ein entsprechendes Ausgangssignal UF bzw. UFR der Flussraten - Messeinrichtung des Dialysators in die Berechnung mit ein. Auch dieses Ausgangssignal kann zu wenigstens einem ersten Zeitpunkt t1 und einem zweiten Zeitpunkt t2 erfasst werden, und die Dialysedosis wird dann wie vorstehend erläutert durch Vergleich der Dialysierflüssigkeit - Parameter, des Flüssigkeitsentzugs und der Zeitdauer zwischen t1 und t2 ermittelt.

Bei allen Berechnungen mit mehrfacher Erfassung der Dialysierflüssigkeit - Parameter, insbesondere jedoch bei der letztgenannten Art der Bestimmung des Verteilungsvolumens werden die Werte für die Dialysierflüssigkeit - Parameter und den Flüssigkeitsentzug zu dem wenigstens ersten und zweiten Zeitpunkt vorzugsweise mit jeweils einem vorgegebenen Wert gewichtet.

Die vorliegende Erfindung betrifft zusammenfassend eine Vorrichtung zum Bestimmen eines Verteilungsvolumens wenigstens einer urämischen Substanz bei einem Dialysepatienten mit: einem Dialysator, optional einer Flussraten - Messeinrichtung zum Erfassen der Durchflussrate einer Dialysierflüssigkeit durch den Dialysator, einer Dialysierflüssigkeit - Messeinrichtung zum Erfassen wenigstens eines Dialysierflüssigkeit - Parameters, der von der wenigstens einen Substanz in der Dialysierflüssigkeit abhängt und einer Rechnereinrichtung zum Ermitteln einer Dialysedosis aus dem Dialysierflüssigkeit - Parameter, und zum Ermitteln des Verteilungsvolumens aus der Dialysedosis unter Berücksichtigung eines Clearance-Faktors und einer Dialysedauer.

Die Rechnereinrichtung zum Ermitteln der Dialysedosis und des Verteilungsvolumens kann sowohl als eine einzelne Rechnereinrichtung ausgebildet sein, aber auch aus mehreren Rechnereinrichtungen bestehen. Darüber hinaus kann/können die Rechnereinrichtung(en) in die Dialysierflüssigkeit - Messeinrichtung integriert sein.

Zusammenfassen betrifft die vorliegende Erfindung eine Vorrichtung und ein Steuerverfahren zum Bestimmen eines Verteilungsvolumens wenigstens einer urämischen Substanz bei einem Dialysepatienten mit:

einem Dialysator,

optional einer Flussraten - Messeinrichtung zum Erfassen der Durchflussrate einer Dialysierflüssigkeit durch den Dialysator,

einer Dialysierflüssigkeit - Messeinrichtung zum Erfassen wenigstens eines Dialysierflüssigkeit - Parameters, der von der wenigstens einen Substanz in der Dialysierflüssigkeit abhängt, und

einer Rechnereinrichtung zum Ermitteln einer Dialysedosis aus dem Dialysierflüssigkeit - Parameter, und zum Ermitteln des Verteilungsvolumens aus der Dialysedosis unter Berücksichtigung eines Clearance-Faktors und einer Dialysedauer.

## Patentansprüche

1. Vorrichtung zum Bestimmen eines Verteilungsvolumens wenigstens einer urämischen Substanz bei einem Dialysepatienten mit:

   - einem Dialysator zum Entziehen der wenigstens einen Substanz aus einer Körperflüssigkeit, der dafür angepasst ist von einer Dialysierflüssigkeit zum Entsorgen der wenigstens einen entzogenen Substanz durchströmt zu werden

   - optional einer Flussraten - Messeinrichtung zum Erfassen einer Durchflussrate der Dialysierflüssigkeit durch den Dialysator,

   - einer Dialysierflüssigkeit - Messeinrichtung zum Erfassen wenigstens eines Dialysierflüssigkeit - Parameters, der von der wenigstens einen Substanz in der Dialysierflüssigkeit abhängt, und

   - mindestens einer Rechnereinrichtung, die konfiguriert ist zum verzögerungsfreien Ermitteln einer Dialysedosis aus dem Dialysierflüssigkeit - Parameter und zum Ermitteln des Verteilungsvolumens aus der Dialysedosis unter Berücksichtigung eines Clearance-Faktors und einer Dialysedauer,

   wobei die Dialysedosis (D) sowie die Clearance (K) und die Dauer der Dialyse, d.h. das Zeitintervall (t) bekannt sind, wobei die Bestimmung der Dialysedosis (D) ohne implizite Modellannahmen auskommt, d.h. kein Verteilungsvolumen theoretisch vorausgesetzt wird, um die Dialysedosis (D) zu bestimmen, wobei der Flüssigkeitsentzug (UF, Ultrafiltration) in der Berechnung Berücksichtigung findet:

   $$K *t \ / \ (0{,}5 * [V1 + \{V1 - UF\}]),$$

   wobei V1 das Volumen zu Beginn des Messzeitraums ist, wobei durch die verzögerungsfreie Bestimmung der Dialysedosis (D) zu jedem Zeitpunkt während der Therapie das aktuelle Harnstoffverteilungsvolumen des Patienten bestimmbar ist.

2. Vorrichtung nach Anspruch 1, bei der die Rechnereinrichtung die Dialysedosis aus einer gemessenen optischen Extinktion der Dialysierflüssigkeit ermittelt.

3. Vorrichtung nach Anspruch 1, bei der die Rechnereinrichtung die Dialysedosis aus einer gemessenen Leitfähigkeit der Dialysierflüssigkeit ermittelt.

4. Vorrichtung nach Anspruch 1, bei der die Rechnereinrichtung die Dialysedosis aus einer erfassten chemischen Reaktion (Urease) ermittelt.

5. Vorrichtung nach einem der vorangehenden Ansprüche, bei der die Rechnereinrichtung die Dialysedosis in Abhängigkeit von dem Dialysierflüssigkeit - Parameter zu einem ersten Zeitpunkt t1 und wenigstens zu einem zweiten Zeitpunkt t2 ermittelt.

6. Vorrichtung nach Anspruch 5, bei der die Rechnereinrichtung die Dialysedosis als Mittelwert von wenigstens zwei Dialysedosis-Werten ermittelt.

7. Vorrichtung nach einem der vorangehenden Ansprüche, bei der die Rechnereinrichtung die Dialysedosis in Abhängigkeit von dem Dialysierflüssigkeit - Parameter und von einem Flüssigkeitsentzug des Dialysepatienten oder aus einem Vergleich zwischen dem Dialysierflüssigkeits-Eingangsfluss und dem Dialysierflüssigkeits-Ausgangsfluss vorzugsweise am Dialysator ermittelt, wobei der Flüssigkeitsentzug aus der Durchflussrate der Dialysierflüssigkeit durch den Dialysator ermittelbar ist.

8. Vorrichtung nach Anspruch 7, bei der die Dialysierflüssigkeit - Messeinrichtung den Dialysierflüssigkeit - Parameter und die Flussraten - Messeinrichtung den Flüssigkeitsentzug zu wenigstens einem ersten Zeitpunkt t1 und einem zweiten Zeitpunkt t2 erfasst und die Rechnereinrichtung das Verteilungsvolumen durch Vergleich der Dialysierflüssigkeit - Parameter, des Flüssigkeitsentzugs und der Zeitdauer zwischen t1 und t2 ermittelt.

9. Vorrichtung nach Anspruch 8, bei der die Rechnereinrichtung die Werte für den Dialysierflüssigkeit - Parameter und den Flüssigkeitsentzug zu dem wenigstens ersten und zweiten Zeitpunkt mit jeweils einem vorgegebenen Wert wichtet.

**Claims**

1. An apparatus for determining a distribution volume of at least one uremic substance in a dialysis patient, comprising:

- a dialyser for withdrawing the at least one substance from a body fluid which is adapted for dialysis fluid flowing through the same in order to dispose of the at least one withdrawn substance,
- optionally, a flow rate measuring system for detecting a flow rate of the dialysis fluid through the dialyser,
- a dialysis fluid measuring system for detecting at least one dialysis fluid parameter that depends on the at least one substance in the dialysis fluid, and
- at least one computer configured for instantaneously establishing a dialysis dose based on the dialysis fluid parameter and for establishing the distribution volume based on the dialysis dose taking a clearance factor and dialysis duration into account,

wherein the dialysis dose (D) as well as the clearance (K) and the dialysis duration, i.e. the time interval (t), are known,

wherein the determination of the dialysis dose (D) manages without implied model assumptions, i.e. that no distribution volume is theoretically provided so as to determine the dialysis dose (D),

wherein the fluid withdrawal (UF, ultrafiltration) is taken into account in the calculation:

$$K * t / (0.5 * [V1 + \{V1 - UF\}]),$$

wherein V1 is the volume at the beginning of the measuring period,

wherein by instantaneously determining the dialysis dose (D) at any point in time during therapy, the current urea distribution volume of the patient can be determined.

2. The apparatus according to claim 1, wherein the computer establishes the dialysis dose based on measured optical extinction of the dialysis fluid.

3. The apparatus according to claim 1, wherein the computer establishes the dialysis dose based on measured conductivity of the dialysis fluid.

4. The apparatus according to claim 1, wherein the computer establishes the dialysis dose based on a detected chemical reaction (urease).

5. The apparatus according to any one of the preceding claims, wherein the computer establishes the dialysis dose in response to the dialysis fluid parameter at a first time t1 and at least at a second time t2.

6. The apparatus according to claim 5, wherein the computer establishes the dialysis dose as mean value of at least two dialysis dose values.

7. The apparatus according to any one of the preceding claims, wherein the computer establishes the dialysis dose in response to the dialysis fluid parameter and to a fluid withdrawal of the dialysis patient or based on a comparison between the dialysis fluid inlet flow and the dialysis fluid outlet flow preferably at the dialyser, wherein the fluid withdrawal can be established based on the flow rate of the dialysis fluid through the dialyser.

8. The apparatus according to claim 7, wherein the dialysis fluid measuring system detects the dialysis fluid parameter and the flow rate measuring system detects the fluid withdrawal at least at a first time t1 and a second time t2 and the computer establishes the distribution volume by comparison of the dialysis fluid parameter, the fluid withdrawal and the time interval between t1 and t2.

9. The apparatus according to claim 8, wherein the computer weights the values for the dialysis fluid parameter and the fluid withdrawal at the at least first and second times with a respective predetermined value.


**Revendications**

1. Dispositif de détermination d'un volume de distribution d'au moins une substance urémique pour un patient sous dialyse avec :

    - un dialyseur pour le prélèvement d'au moins une substance d'un liquide corporel qui est adapté afin d'être parcouru par un liquide de dialyse pour l'élimination de l'au moins une substance prélevée,
    - en option un dispositif de mesure de débit pour la détection d'un débit du liquide de dialyse par le dialyseur,
    - un dispositif de mesure de liquide de dialyse pour la détection d'au moins un paramètre de liquide de dialyse qui dépend de l'au moins une substance dans le liquide de dialyse, et
    - au moins un dispositif informatique qui est configuré pour déterminer sans latence une dose de dialyse à partir du paramètre de liquide de dialyse et pour déterminer le volume de distribution à partir de la dose de dialyse en tenant compte d'un facteur de clairance et d'une durée de dialyse,

    dans lequel la dose de dialyse (D) ainsi que la clairance (K) et la durée de la dialyse c'est-à-dire l'intervalle de temps (t) sont connues,
    dans lequel la détermination de la dose de dialyse (D) ne comporte pas d'hypothèses de modèle implicites, c'est-à-dire aucun volume de distribution n'est présupposé théoriquement afin de déterminer la dose de dialyse (D),
    dans lequel le prélèvement de liquide (UF, ultrafiltration) est pris en considération dans le calcul :

$$K*t \, / \, (0{,}5 * [V1 + \{V1 - UF\}]),$$

    dans lequel V1 est le volume au début de la période de mesure,
    dans lequel le volume de distribution d'urée actuel du patient peut être déterminé par la détermination sans latence de la dose de dialyse (D) à chaque moment pendant le traitement.

2. Dispositif selon la revendication 1, pour lequel le dispositif informatique détermine la dose de dialyse à partir d'une extinction optique mesurée du liquide de dialyse.

3. Dispositif selon la revendication 1, pour lequel le dispositif informatique détermine la dose de dialyse à partir d'une conductivité mesurée du liquide de dialyse.

4. Dispositif selon la revendication 1, pour lequel le dispositif informatique détermine la dose de dialyse à partir d'une réaction chimique détectée (uréase).

5. Dispositif selon l'une des revendications précédentes, pour lequel le dispositif informatique détermine la dose de dialyse en fonction du paramètre de liquide de dialyse à un premier moment t1 et au moins à un second moment t2.

**6.** Dispositif selon la revendication 5, pour lequel le dispositif informatique détermine la dose de dialyse comme valeur moyenne d'au moins deux valeurs de dose de dialyse.

**7.** Dispositif selon l'une des revendications précédentes, pour lequel le dispositif informatique détermine la dose de dialyse en fonction du paramètre de liquide de dialyse et d'un prélèvement de liquide du patient de dialyse ou d'une comparaison entre le flux d'entrée de liquide de dialyse et le flux de sortie de liquide de dialyse de préférence sur le dialyseur, dans lequel le prélèvement de liquide peut être déterminé à partir du débit du liquide de dialyse au travers du dialyseur.

**8.** Dispositif selon la revendication 7, pour lequel le dispositif de mesure de liquide de dialyse détecte le paramètre de liquide de dialyse et le dispositif de mesure de débit détecte le prélèvement de liquide à au moins un premier moment t1 et à un second moment t2 et le dispositif informatique détermine le volume de distribution par comparaison du paramètre de liquide de dialyse, du prélèvement de liquide et de la durée entre t1 et t2.

**9.** Dispositif selon la revendication 8, pour lequel le dispositif informatique pondère les valeurs pour le paramètre de liquide de dialyse et le prélèvement de liquide à l'au moins un premier et second moment avec respectivement une valeur prescrite.

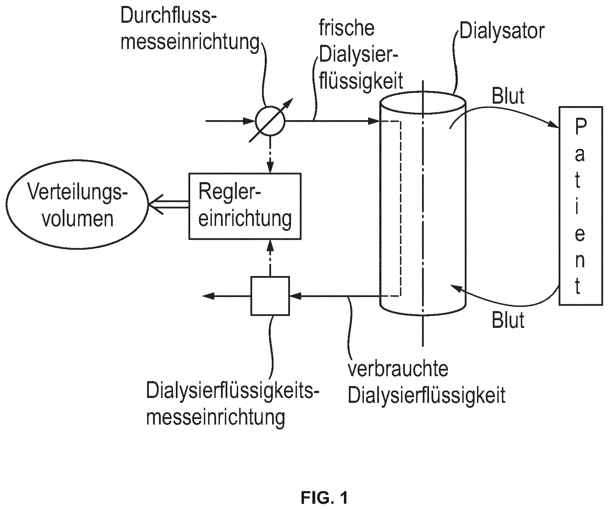

**FIG. 1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20070027402 A1
- WO 2010043381 A2
- EP 1037681 B1
- EP 0986410 B1
- DE 69834034 T2
- WO 9855166 A1
- US 7077819 B1

**In der Beschreibung aufgeführte Nicht-Patentliteratur**

- **LOPOT et al.** Continuous blood volume monitoring and dry weight assessment. *J Ren Care,* April 2007, vol. 33 (2), 52-8
- **RODRIGUEZ et al.** Assessment of dry weight by monitoring changes in blood volume during hemodialysis using Crit-Line. *Kidney International,* 2005, vol. 68, 854-861